(19) Europäisches Patentamt
European Patent Office
Office européen des brevets

(11) Veröffentlichungsnummer: **0 417 626 A2**

(12) **EUROPÄISCHE PATENTANMELDUNG**

(21) Anmeldenummer: **90117057.1**

(22) Anmeldetag: **05.09.90**

(51) Int. Cl.5: **C07C 257/06**, C07C 323/48, A01N 37/52

(30) Priorität: **09.09.89 DE 3930112**

(43) Veröffentlichungstag der Anmeldung: **20.03.91 Patentblatt 91/12**

(84) Benannte Vertragsstaaten: **DE ES FR GB IT**

(71) Anmelder: **HOECHST AKTIENGESELLSCHAFT**
**Postfach 80 03 20**
**W-6230 Frankfurt am Main 80(DE)**

(72) Erfinder: **Löher, Heinz Josef, Dr.**
**Amselweg 9**
**W-6238 Hofheim am Taunus(DE)**
Erfinder: **Sohn, Erich, Dr.**
**Lange Gasse 4**
**W-8900 Augsburg(DE)**
Erfinder: **Bauer, Klaus, Dr.**
**Doorner Strasse 53d**
**W-6450 Hanau(DE)**
Erfinder: **Bieringer, Hermann, Dr.**
**Eichweg 26**
**W-6239 Eppstein/Taunus(DE)**

# (54) N-Arylhydrazone enthaltende pflanzenschützende Mittel und neue N-Arylhydrazone.

(57) Die Verbindungen der Formel (I) sind geeignet, phytotoxische Nebenwirkungen von Herbiziden an Kulturpflanzen zu reduzieren oder zu unterbinden.

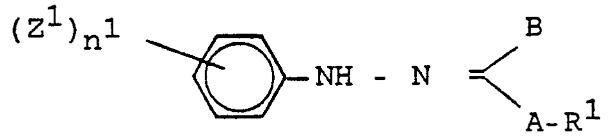

(I)

Dabei bedeuten

$Z^1$ Hal, $NO_2$, CN, R, RO, RS, cyclo-R, wobei R = Alkyl oder durch Alkoxy, OH oder Halogen substituiertes Alkyl ist, $NH_2$, RNH, RRN, Phenyl, Phenoxy, Halogenphenyl, Halogenphenoxy,

$R^1$ R, cyclo-R, wobei R gesättigt oder ungesättigtes und/oder durch R'O, R'S, R'NH, R'R'N, R'OCO, R'COO, CN oder Halogen substituierte Alkyl ist, Phenylalkyl, gegebenenfalls substituiertes Phenyl,

$n^1$ 0 - 5, A Sauerstoff oder Schwefel,

B $-CO-R^2$ oder Phenyl-$(Z^2)_n2$, worin

$Z^2$ wie $Z^1$ definiert ist und $n^2$ 0 bis 5 ist,

$R^2$ OH, RO, RS, cyclo-RO, wobei R = gesättigt oder ungesättigt und/oder durch R'O, R'S, R'N, R'R'N, CN oder Halogen substituiertes Alkyl ist, $RRRSiCH_2O$, einen Rest der Formel -NR-Phenyl-$(Z^3)_n3$ (R = H, Alkyl) 2,3-Oxazolin-2-yl, gegebenenfalls alkylsubstituiert,

$Z^3$ wie $Z^1$ definiert, $n^3$ = 0 - 5, Mono- oder Dialkylamino, Cycloalkylamino, Pyridino, Morpholino, Dimethylmorpholino, einen Rest der Formel $-O-N=CR^3R^4$, worin $R^3$ und $R^4$ Alkylreste oder zusammen mit dem C-Atom einen Cycloalkylrest bedeuten.

EP 0 417 626 A2

## N-ARYLHYDRAZONE ENTHALTENDE PFLANZENSCHÜTZENDE MITTEL UND NEUE N-ARYLHYDRAZONE

Die Erfindung betrifft Safener oder Antidote, die in Kombination mit Herbiziden die Phytotoxizität der Herbizide bei Kulturpflanzen herabsetzen können.

Gegenstand der Erfindung sind pflanzenschützende Mittel, welche N-Arylhydrazone der allgemeinen Formel (I) oder deren Salze

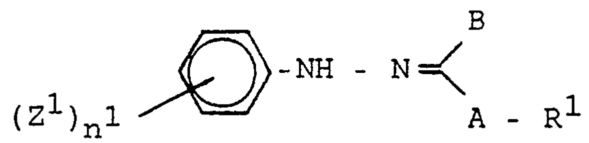

(I),

worin

$Z^1$ Halogen, Nitro, Cyano oder Alkyl, Alkoxy, Alkylthio oder Cycloalkyl, wobei die letzten vier genannten Reste unsubstituiert oder ein- oder mehrfach durch Alkoxy, Hydroxy oder Halogen substituiert sind, ferner Amino, Mono- oder Dialkylamino, Phenyl oder Phenoxy, wobei Phenyl oder Phenoxy unsubstituiert oder ein- oder mehrfach durch Halogen oder Halogenalkyl substituiert ist,

$R^1$ Alkyl, Alkenyl, Alkinyl oder Cycloalkyl, wobei die letztgenannten vier Reste unsubstituiert oder ein- oder mehrfach durch Reste aus der Gruppe Alkoxy, Alkylthio, Mono- oder Dialkylamino, Alkoxycarbonyl, Alkylcarbonyloxy, Cyano und Halogen substituiert sind, ferner Phenylalkyl oder Phenyl, die jeweils im Phenylrest unsubstituiert oder ein- oder mehrfach durch Reste aus der Gruppe Alkyl, Alkoxy, Alkylthio, Mono- und Dialkylamino, Alkoxycarbonyl, Cyano und Halogen substituiert sind,

$n^1$ eine ganze Zahl von 0 bis 5 ist,

A O oder S,

B einen Rest der Formel

$-CO-R^2$ oder

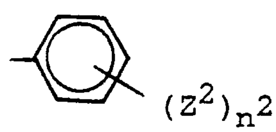

,

worin

$Z^2$ die für $Z^1$ genannte Bedeutung hat und

$n^2$ eine ganze Zahl von 0 bis 5 ist, und

$R^2$ OH oder Alkoxy, Alkenyloxy, Alkinyloxy, Alkylthio oder Cycloalkoxy, wobei die fünf letztgenannten Reste unsubstituiert oder ein- oder mehrfach durch Reste aus der Gruppe Alkoxy, Alkylthio, Mono- und Dialkylamino, Cyano und Halogen substituiert sind,

ferner Trialkylsilylmethoxy, einen Rest der Formel

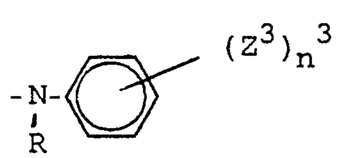

oder

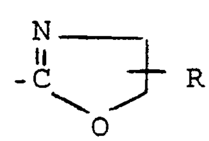

,

worin jeweils R Wasserstoff oder Alkyl bedeutet, $Z^3$ die für $Z^1$ genannte Bedeutung hat und $n^3$ eine ganze Zahl von 0 bis 5 ist,

ferner Mono- oder Dialkylamino, Cycloalkylamino, Pyridino, Morpholino, Dimethylmorpholino, einen Rest der Formel

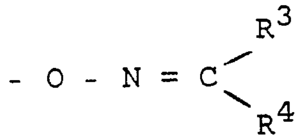
,

worin $R^3$ und $R^4$ unabhängig voneinander Alkylreste bedeuten oder $R^3$ und $R^4$ gemeinsam mit dem sie verknüpfenden C-Atom einen Cycloalkylrest bilden,
bedeuten,
in Kombination mit üblichen Hilfsstoffen für Pflanzenschutzmittelformulierungen enthalten.

In der Formel (I) können Alkyl, Alkoxy- und Alkylthioreste sowie die entsprechenden ungesättigten Reste jeweils geradkettig oder verzweigt sein. Halogen bedeutet Fluor, Chlor, Brom oder Jod, vorzugsweise Fluor, Chlor oder Brom, insbesondere Fluor oder Chlor.

Im Falle $R^2$ = OH können die Verbindungen der Formel (I) Salze bilden. Erfindungsgemäß einsetzen lassen sich die in der Landwirtschaft verwendbaren Salze. Als solche kommen beispielsweise Metallsalze wie Alkali- oder Erdalkalisalze, insbesondere Natrium- oder Kaliumsalze, Salze mit Ammonium, Mono-, Di-, Tri- oder Tetra-($C_1$-$C_4$)alkylammonium oder mit Mono-, Di-, Tri- oder Tetra-($C_1$-$C_4$)alkanolammonium infrage.

Ferner erfaßt Formel (I) auch alle Stereoisomeren und deren Gemische. Stereoisomere können vor allem auftreten, wenn ein asymmetrisches C-Atom oder geeignet substituierte Doppelbindungen in der Formel (I) vorhanden sind.

Von besonderem Interesse sind erfindungsgemäße pflanzenschützende Mittel mit N-Arylhydrazonen der Formel (I) oder deren Salzen,
worin
$Z^1$ unabhängig voneinander Halogen, Nitro, Cyano, ($C_1$-$C_4$)Alkyl, ($C_1$-$C_4$)Alkoxy, ($C_1$-$C_4$)Alkylthio, wobei die Alkyl-, Alkoxy- und Alkylthiogruppen unsubstituiert oder ein-oder mehrfach durch Halogenatome, insbesondere Fluor oder Chlor substituiert sind, ($C_3$-$C_6$)Cycloalkyl, das unsubstituiert oder durch ($C_1$-$C_4$)-Alkyl substituiert ist, Amino, ($C_1$-$C_4$)Alkylamino, Di-($C_1$-$C_4$)alkylamino, Hydroxymethyl, ($C_1$-$C_4$)Alkoxy-methyl, Phenyl oder Phenoxy, wobei Phenyl und Phenoxy unsubstituiert oder ein- oder mehrfach durch Halogen oder einfach durch Trifluormethyl oder durch ein oder mehrere Halogen und ein Trifluormethyl substituiert ist,
$n^1$ 0, 1, 2 oder 3,
A S oder O,
$R^1$ ($C_3$-$C_6$)Cycloalkyl, ($C_1$-$C_6$)Alkyl, ($C_2$-$C_6$)Alkenyl, ($C_2$-$C_6$)Alkinyl, wobei die genannten vier Reste unsubstituiert oder ein- oder mehrfach durch Reste aus der Gruppe ($C_1$-$C_2$)Alkoxy, Mono- oder Di-($C_1$-$C_6$)-Alkylamino, ($C_1$-$C_6$)Alkoxycarbonyl, ($C_1$-$C_6$)Alkylcarbonyloxy, ($C_1$-$C_2$)Alkylthio, Cyano und Halogen substituiert sind, ferner Phenyl-($C_1$-$C_6$)alkyl oder Phenyl, die jeweils im Phenylrest unsubstituiert oder ein- oder mehrfach durch Reste aus der Gruppe ($C_1$-$C_4$)Alkyl, ($C_1$-$C_4$)Alkoxy, ($C_1$-$C_4$)Alkylthio, Mono- und Di-($C_1$-$C_6$)-alkylamino, ($C_1$-$C_6$)Alkoxy-carbonyl, Cyano, und Halogen substituiert sind,
B einen Rest der Formel
$-CO-R^2$ oder

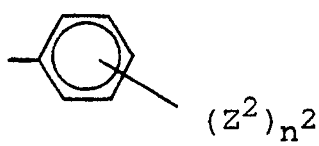
,

worin
$Z^2$ die oben für $Z^1$ genannte Bedeutung hat und $n^2$ 0, 1, 2 oder 3 ist,
$R^2$ Hydroxy, $-OCH_2Si(CH_3)_3$, ($C_3$-$C_6$)Cycloalkoxy, Phenyl($C_1$-$C_6$)alkoxy, Phenoxy, ($C_2$-$C_6$)Alkenyloxy, ($C_2$-$C_6$)Alkinyloxy, ($C_1$-$C_6$)Alkoxy, ($C_1$-$C_6$)Alkylthio, wobei die Alkoxy oder Alkylthiogruppe unsubstituiert oder ein- oder zweifach durch ($C_1$-$C_2$)Alkoxy, Mono- oder Di-($C_1$-$C_6$)Alkylamino, ($C_1$-$C_2$)Alkylthio, Cyano oder ein-oder mehrfach durch Halogen substituiert ist,
einen Rest der Formel

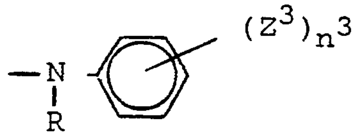 oder 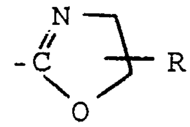 ,

worin jeweils R Wasserstoff oder $(C_1-C_4)$Alkyl bedeutet,
$Z^3$ die für $Z^1$ genannte Bedeutung hat und $n^3$ 0, 1, 2 oder 3 ist,
ferner Mono- oder Di-$(C_1-C_4)$Alkylamino, $(C_5-C_6)$Cycloalkylamino, Piperidino, Morpholino oder 2,6-Dimethylmorpholino, einen Rest der Formel

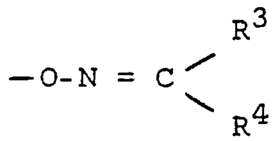 ,

worin
$R^3$ und $R^4$ unabhängig voneinander $(C_1-C_4)$Alkyl bedeuten oder $R^3$ und $R^4$ gemeinsam mit dem sie verknüpfenden C-Atom einen 5-, 6- oder 7-gliedrigen Cycloalkylrest bilden,
bedeuten.

Bevorzugt sind erfindungsgemäße pflanzenschützende Mittel mit N-Arylhydrazonen der Formel (I) oder deren Salzen,
worin
$Z^1$ unabhängig voneinander Halogen, insbesondere Fluor oder Chlor, Nitro, $(C_1-C_4)$Alkyl, $(C_1-C_4)$Alkoxy oder Trifluormethyl,
A Sauerstoff,
$n^1$ 0, 1 oder 2,
$R^1$ Phenyl, das unsubstituiert oder ein- bis dreifach durch Reste aus der Gruppe $(C_1-C_4)$Alkyl, insbesondere Methyl, $(C_1-C_4)$Alkoxy und Halogen substituiert ist, oder $(C_1-C_4)$Alkyl,
B einen Rest der Formel
-CO - $R^2$ oder

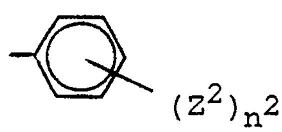 ,

worin
$R^2$ Hydroxy oder $(C_1-C_4)$Alkoxy,
$Z^3$ Halogen, insbesondere Chlor, oder $(C_1-C_4)$Alkyl und
$n^3$ 0, 1 oder 2 sind,
bedeuten.

Einige der Verbindungen der Formel (I) sind aus wissenschaftlichen Veröffentlichungen (Bull. Chem. Soc. Japan 48 , 365-366 (1975), J. Chem. Soc. (C) 968 (1970); Tetrahedron 28 , 5903 (1972); Tetrahedron Lett. 31 , 3211 (1972); Can. J. Chem. 91 , 4115 (1973)) bekannt. Ihre Safener-Wirkung sind jedoch bisher nicht erkannt worden.

Gegenstand der vorliegenden Erfindung sind daher auch die bisher nicht beschriebenen neuen Verbindungen der Formel (I) oder deren Salze. Es sind dies die oben definierten Verbindungen der Formel (I) oder deren Salze mit Ausnahme der Verbindungen, bei denen
a) $R^1$ unsubstituiertes oder substituiertes Phenyl,
A Sauerstoff und
B einen Rest der oben definierten Formel

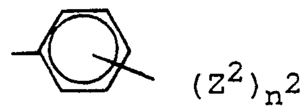

bedeuten, sowie mit Ausnahme solcher, bei denen

b) $Z^1$ 4-$CH_3$, 4-Br, 4-Cl, 4-$NO_2$, 3-$CH_3$,

$n^1$ 0 oder 1,

$R^1$ unsubstituiertes Phenyl, 4-Methylphenyl oder 3-Methylphenyl,

A Sauerstoff und

B $CO_2C_2H_5$

bedeuten.

Gegenstand der vorliegenden Erfindung ist auch ein Verfahren zur Herstellung der neuen Verbindungen der Formel (I) und deren Salze, dadurch gekennzeichnet, daß man eine Verbindung der Formel (II)

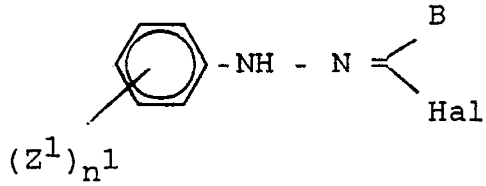

(II),

worin $(Z)_n1$ und B die oben angegebenen Bedeutungen besitzen und Hal Br, Cl, J ist,
mit Alkoholen bzw. Thiolen der Formel HA-$R^1$
worin A und $R^1$ die oben genannten Bedeutungen haben,
und einer Base, wie z.B. NaOEt, $ROC_2H_5$, $NH_3$ und $NaOCOCH_3$,
umsetzt.

Als Lösungsmittel eignen sich unpolare organische Lösungsmittel, z.B. Ether wie Diethylether oder THF, oder die zur Umsetzung erforderlichen Alkohole selbst.

Die Verbindungen der Formel (II) sind literaturbekannt (z.B. Bull. Chem. Soc. Jpn 48 365-366 (1975)) oder lassen sich analog den bekannten Verbindungen herstellen.

Die Verbindungen der Formel (I) reduzieren oder unterbinden phytotoxische Nebenwirkungen von Pflanzenschutzmitteln, insbesondere von Herbiziden, die bei deren Einsatz in Nutzpflanzenkulturen auftreten können.

Die Verbindungen der Formel (I) können nacheinander oder zusammen mit den herbiziden Wirkstoffen ausgebracht werden. Sie sind dann in der Lage, schädliche Nebenwirkungen der Herbizide bei Kulturpflanzen zu vermindern oder völlig aufzuheben, ohne die Wirksamkeit dieser Herbizide gegen Schadpflanzen zu beeinträchtigen.

Hierdurch kann das Einsatzgebiet herkömmlicher Pflanzenschutzmittel ganz erheblich erweitert werden. Solche Verbindungen, die die Eigenschaft besitzen, Kulturpflanzen gegen phytotoxische Schäden durch Herbizide zu schützen, werden "Antidote" oder "Safener" genannt.

Herbizide, deren phytotoxische Nebenwirkungen mittels der Verbindungen der Formel (I) herabgesetzt werden können, sind z.B. Carbamate, Thiocarbamate, Halogenacetanilide, substituierte Phenoxy-, Naphthoxy- und Phenoxyphenoxycarbonsäurederivate sowie Heteroaryloxyphenoxycarbonsäurederivate wie Chinolyloxy-, Chinoxalyloxy-, Pyridyloxy-, Benzoxazolyloxy-, Benzthiazolyloxy-phenoxy-carbonsäureester und ferner Dimedonoximabkömmlinge. Bevorzugt hiervon sind Phenoxyphenoxy- und Heteroaryloxyphenoxycarbonsäureester. Als Ester kommen hierbei insbesondere niedere Alkyl-, Alkenyl und Alkinylester in Frage.

Beispielsweise seien, ohne daß dadurch eine Beschränkung erfolgen soll, folgende Herbizide genannt:

A) Herbizide vom Typ der Phenoxyphenoxy- und Heteroaryloxyphenoxycarbonsäure-($C_1$-$C_4$)alkyl-, -($C_2$-$C_4$)alkenyl- und -($C_3$-$C_4$)alkinylester wie 2-(4-(2,4-Dichlorphenoxy)-phenoxy)-propionsäuremethylester, 2-(4-(4-Brom-2-chlorphenoxy)-phenoxy)-propionsäuremethylester, 2-(4-(4-Trifluormethylphenoxy)-phenoxy)-propionsäuremethylester, 2-(4-(2-Chlor-4-trifluormethylphenoxy)-phenoxy)-propionsäuremethylester, 2-(4-(2,4-Dichlorbenzyl)-phenoxy)-propionsäuremethylester, 4-(4-(4-Trifluormethylphenoxy)-phenoxy)-pent-2-en-säureethylester, 2-(4-(3,5-Dichlorpyridyl-2-oxy)-phenoxy)-propionsäureethylester, 2-(4-(3,5-Dichlorpyridyl-2-oxy)-phenoxy)-propionsäurepropargylester, 2-(4-(6-Chlorbenzoxazol-2-yl-oxy)-phenoxy)-propionsäureethylester, 2-(4-(3-Chlor-5-trifluormethyl-2-pyridyloxy)-phenoxy)-propionsäuremethylester, 2-(4-(5-Trifluormethyl-2-pyridyloxy)-phenoxy)-propionsäurebutylester, 2-(4-(3-Fluor-5-chlorpyridyl-2-oxy)-phenoxy)-propionsäuremethylester, 2-(4-(3-Fluor-5-chlorpyridyl-2-oxy)-phenoxy)-propionsäurepropargylester, 2-(4-(6-Chlor-2-chinoxalyloxy)phenoxy)-propionsäuremethylester, 2-(4-(6-Fluor-2-chinoxalyloxy)-phenoxy)-propionsäuremethylester, 2-(4-(6-Chlor-2-chinolyloxy)-phenoxy)-propionsäuremethylester,
B) Chloracetanilid-Herbizide wie N-methoxymethyl-2,6-diethyl-chloracetanilid, N-(3′-Methoxyprop-2′-yl)-methyl-6-ethyl-chloracetanilid, N-(3-methyl-1,2,4-oxdiazol-5-yl-methyl)-chloressigsäure-2,6-dimethylanilid,
C) Thiocarbamate wie S-Ethyl-N,N-dipropylthiocarbamat oder S-Ethyl-N,N-diisobutylthiocarbamat
D) Dimedon-Derivate wie Methyl-3-(1-allyloxyimino)butyl-4-hydroxy-6,6-dimethyl-2-oxocyclohex-3-encarboxylat 2-(N-Ethoxybutyrimidoyl)-5-(2-ethylthiopropyl)-3-hydroxy-2-cyclohexen-1-on, 2-(N-Ethoxybutyrimidoyl)-5-(2-phenylthiopropyl)-3-hydroxy-2-cyclohexen-1-on, 2-(1-Allyloxyiminobutyl)-4-methoxycarbonyl-5,5-dimethyl-3-oxocyclohexenol, 2-(1-(3-Chlorallyloxy)-iminobutyl)-5-(2-ethylthio)propyl)-3-hydroxy-cyclohex-2-enon 2-(1-(Ethoxyimino)-butyl)-3-hydroxy-5-(thian-3-yl)-cyclohex-2-enon oder 2-(1-Ethoxyiminopropyl)-5-(2,4,6-trimethylphenyl)-3-hydroxy-2-cyclohexen-1-on.

Das Mengenverhältnis Safener:Herbizid kann innerhalb weiter Grenzen, im Bereich zwischen 1:10 und 10:1, insbesondere zwischen 2:1 und 1:10, schwanken. Die jeweils optimalen Mengen an Herbizid und Safener sind abhängig vom Typ des verwendeten Herbizids oder vom verwendeten Safener sowie von der Art des zu behandelnden Pflanzenbestandes und lassen sich von Fall zu Fall durch entsprechende Versuche ermitteln.

Haupteinsatzgebiete für die Anwendung der Safener sind vor allem Getreidekulturen (Weizen, Roggen, Gerste, Hafer) Reis, Mais, Sorghum, aber auch Baumwolle, Zuckerrübe, Zuckerrohr und Sojabohne.

Die Safener der Formel (I) können je nach ihren Eigenschaften zur Vorbehandlung des Saatgutes der Kulturpflanze (Beizung der Samen) verwendet werden oder vor der Saat in die Saatfurchen eingebracht werden oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Die benötigten Aufwandmengen der Verbindungen der Formel (I) können je nach Indikation und verwendetem Herbizid innerhalb weiter Grenzen schwanken und variieren im allgemeinen zwischen 0,01 und 10 kg Wirkstoff je Hektar.

Gegenstand der vorliegenden Erfindung ist deshalb auch ein Verfahren zum Schutz von Kulturpflanzen vor phytotoxischen Nebenwirkungen von Herbiziden, das dadurch gekennzeichnet ist, daß eine wirksame Menge einer Verbindung der Formel (I) vor, nach oder gleichzeitig mit dem Herbizid appliziert wird.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die Verbindungen der Formel (I) oder deren Kombination mit einem oder mehreren der genannten Herbizide bzw. Herbizidgruppen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), wäßrige Lösungen (SL), konzentrierte Emulsionen wie Öl-in-Wasser-und Wasser-in-Öl-Emulsionen (EW), versprühbare Lösungen oder Emulsionen, Dispersionen auf Öl- oder Wasserbasis (SC), Stäubemittel (DP), Beizmittel, Boden- bzw. Streugranulate (FG), wasserdispergierbare Granulate (WG), ULV-Formulierungen, Mikrokapseln oder Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Valkenburg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Intruduction to Clay Colloid Chemistry"; 2nd Ed., J. Wiley & Sons, N.Y., Marsh, "Solvents Guide", 2nd Ed., Interscience, N.Y. 1950; McCutcheon's, "Detergents and Emulsifiers Annual", MC Publ. Corp., Ridgewood N.J.; Sisley

and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technolgie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs-oder Inertstoff noch Netzmittel, z.B. polyoxethylierte Alkylphenole, polyoxethylierte Fettalkohole, Alkansulfonate oder Alkylarylsulfonate und Dispergiermittel, z.B. ligninsulfonsaures Natrium, 2,2′-dinaphthylmethan-6,6′-disulfonsaures Natrium, dibutylnaphthalin-sulfonsaures Natrium oder auch oleoylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid- Kondensationsprodukte (z.B. Blockpolymerisate) Alkylpolypolyglykolether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophyllit oder Diatomeenerde.

Boden- bzw. Streu-Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0.1 bis 99 Gewichtsprozent, insbesondere 0,1 bis 95 Gew.-%, Wirkstoff der Formel (I), oder Wirkstoffgemisch Antidot/Herbizid, 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 80 Gew.-%, vorzugsweise 5 bis 80 Gew.-% betragen. Staubförmige Formulierungen enthalten meistens 1 bis 25 Gew.-%, vorzugsweise 5 bis 20 Gew.-% an Wirkstoff, versprühbare Lösungen etwa 0,2 bis 25 Gew.-%, vorzugsweise 2 bis 20 Gew.% Wirkstoff. Bei wasserdispergierbaren Granulaten hängt der Wirkstoffgehalt zum Teil davon ab, ob die wirksame Verbindung flüssig oder fest vorliegt. Im allgemeinen liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate, sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids u.a. variiert die erforderliche Aufwandmenge der Verbindungen der Formel (I). Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Folgende Beispiele dienen zur Erläuterung der Erfindung:

## A. Formulierungsbeispiele

a) Ein Stäubmittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277° C) mischt und in einer Reibkugelmühle auf eine Feinheit von unter 5 Mikron vermahlt.

d) Ein emulgierbares Konzentrat wird erhalten aus 15 Gew.Teilen einer Verbindung der Formel (I), 75 Gew.-Teilen Cyclohexanon als Lösemittel und 10 Gew.-Teilen oxethyliertes Nonylphenol als Emulgator.

e) Ein in Wasser dispergierbares Granulat wird erhalten indem man
75 Gewichtsteile einer Verbindung der Formel I,
10 Gewichtsteile ligninsulfonsaures Calcium,
5 Gewichtsteile Natriumlaurylsulfat,
3 Gewichtsteile Polyvinylalkohol und
7 Gewichtsteile Kaolin
mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man
25 Gewichtsteile einer Verbindung der Formel (I),
5 Gewichtsteile 2,2′-Dinaphthylmethan-6,6′-disulfonsaures Natrium,
2 Gewichtsteile oleoylmethyltaurinsaures Natrium,
1 Gewichtsteil Polyvinylalkohol,
17 Gewichtsteile Calciumcarbonat und
50 Gewichtsteile Wasser
auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet.

**B. Chemische Beispiele**

**2-(4-Methylphenyloxy)-2-(4-nitrophenylhydrazono)-essigsäureethylester (Beispiel Nr. 42)**

0,69 g Natrium werden in 100 ml absolutem Ethanol gelöst. Dazu gibt man 3,24 g 4-Methylphenol und rührt 15 min bei Raumtemperatur nach. Anschließend wird auf 0° C gekühlt und 8,16 g 2-Chlor-2-(4-nitrophenyl-hydrazono)-essigsäureethylester zugegeben. 18 Stunden wird bei Raumtemperatur nachgerührt. Das Produkt wird abgesaugt und mit Essigsäureethylester ausgerührt. Nach Trocknung werden 7,2 g (70 % d.Th.) Produkt vom Schmelzpunkt 158° C erhalten.

**2-Phenyloxy-2-phenylhydrazono-essigsäureethylester (Beispiel Nr. 7)**

Zu einer Lösung von 0,68 g Natriumethanolat in 40 ml absolutem Ethanol werden 0,94 g Phenol gegeben und 15 min gerührt. Bei 0° C werden anschließend 2,26 g 2-Chlor-2-phenylhydrazono-essigsäureethylester zugegeben und 18 Stunden bei Raumtemperatur nachgerührt. Das Produkt wird abgesaugt und aus Ethanol umkristallisiert. So werden 1,7 g (60 %) Produkt vom Schmelzpunkt 82-83° C erhalten.

**2-Methoxy-2-(2,4-Dichlorphenylhydrazono)-essigsäureethylester (Beispiel Nr. 27)**

Bei 0° C werdenzu 147,8 g 2-chlor-2-(2,4-dichlorphenylhydrazono)-essigsäureethylester in 100 ml Tetrahydrofuran 50,59 g Triethylamin getropft. Nach 15 min bei 0° C werden 40 ml Methanol zugegeben und 6 Stunden bei Raumtemperatur gerührt. Es wird vollständig einrotiert und anschließend an Kieselgel chromatographiert (Laufmittel n-Heptan/Essigester 1:1). So werden 21,82 g (15 % d.Th.) Produkt vom Schmelzpunkt 119-122° C erhalten.

**2-Ethoxy-2-(2,4-dichlorphenylhydrazono)-essigsäureethylester (Beispiel 15)**

29,5 g 2-chlor-2-(2,4-dichlorphenylhydrazono)-essigsäureethylester und 8,2 g Natriumacetat werden in

100 ml 95 %igem Ethanol 5 Stunden unter Rückfluß erhitzt. Das Reaktionsgemisch wird anschließend einrotiert, mit $CH_2Cl_2$ aufgenommen und mit Wasser extrahiert. Die organische Phase wird mit $MgSO_4$ getrocknet und einrotiert. Durch anschließende Säulenchromatographie werden 13,7 g Produkt vom Schmelzpunkt 47° C erhalten.

In der folgenden Tabelle I sind die obengenannten Beispiele zusammen mit weiteren Beispielen aufgeführt, die in analoger Weise hergestellt werden können:

**Tabelle I**

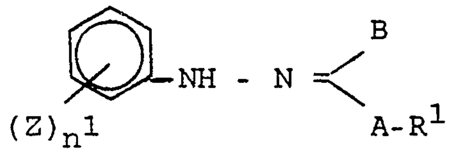

(I)

| Nr. | $(Z)_{n}1$ | $-A-R^1$ | B | Schmp. °C |
|---|---|---|---|---|
| 1 | 2-CL | $-O-C_6H_5$ | $CO_2C_2H_5$ | |
| 2 | 4-Cl | " | " | |
| 3 | $2,4-Cl_2$ | " | " | |
| 4 | $3,5-Cl_2$ | " | " | |
| 5 | $2,6-Cl_2$ | " | " | |
| 6 | $4-NO_2$ | " | " | |
| 7 | H | " | " | 82-83 |
| 8 | $4-OCH_3$ | " | " | |
| 9 | $2-CH_3$ | " | " | |
| 10 | $2,4-(CH_3)_2$ | " | " | |
| 11 | 4-F | " | " | |
| 12 | $3-CF_3$ | " | " | |
| 13 | 2-Cl | $-O-C_2H_5$ | " | |
| 14 | 4-Cl | " | " | |
| 15 | $2,4-Cl_2$ | " | " | 47 |
| 16 | $2,5-Cl_2$ | " | " | |
| 17 | $2,6-Cl_2$ | " | " | |
| 18 | $4-NO_2$ | " | " | |
| 19 | H | " | " | |
| 20 | $4-OCH_3$ | " | " | |
| 21 | $2-CH_3$ | " | " | |
| 22 | $2,4-(CH_3)_2$ | " | " | |
| 23 | 4-F | " | " | |
| 24 | $3-CF_3$ | " | " | |
| 25 | 2-Cl | $-O-CH_3$ | $CO_2C_2H_5$ | |
| 26 | 4-CL | " | " | |
| 27 | $2,4-Cl_2$ | " | " | 119-122 |

(Forts. Tabelle I)

| Nr. | $(Z)_n1$ | $-A-R^1$ | B | Schmp. °C |
|---|---|---|---|---|
| 28 | 3,5-$Cl_2$ | " | " | |
| 29 | 2,6-$Cl_2$ | " | " | |
| 30 | 4-$NO_2$ | " | " | |
| 31 | H | " | " | |
| 32 | 4-$OCH_3$ | " | " | |
| 33 | 2-$CH_3$ | " | " | |
| 34 | 2,4-$(CH_3)_2$ | " | " | |
| 35 | 4-F | " | " | |
| 36 | 3-$CF_3$ | " | " | |
| 37 | 2-Cl | $-O-C_6H_4-CH_3$(p) | " | |
| 38 | 4-Cl | " | " | |
| 39 | 2,4-$Cl_2$ | " | " | |
| 40 | 3,5-$Cl_2$ | " | " | |
| 41 | 2,6-$Cl_2$ | " | " | |
| 42 | 4-$NO_2$ | " | " | |
| 43 | H | " | " | |
| 44 | 4-$OCH_3$ | " | " | |
| 45 | 2-$CH_3$ | " | " | |
| 46 | 2,4-$(CH_3)_2$ | " | " | |
| 47 | 4-F | " | " | |
| 48 | 3-$CF_3$ | " | " | |
| 49 | 2-Cl | $-OC_2H_5$ | $-C_6H_5$ | |
| 50 | 4-Cl | " | " | |
| 51 | 2,4-$Cl_2$ | " | " | |
| 52 | 3,5-$Cl_2$ | " | " | |
| 53 | 2,6-$Cl_2$ | " | " | |
| 54 | 4-$NO_2$ | " | " | |
| 55 | H | " | " | 168-169 |
| 56 | 4-$OCH_3$ | " | " | |
| 57 | 2-$CH_3$ | " | " | |

(Forts. Tabelle I)

| Nr. | $(Z)_n1$ | $-A-R^1$ | B | Schmp. °C |
|---|---|---|---|---|
| 58 | 2,4-$(CH_3)_2$ | $OC_2H_5$ | $-C_6H_5$ | |
| 59 | 4-F | " | " | |
| 60 | 3-$CF_3$ | " | " | |
| 61 | 2-Cl | O-$C_3H_7$(n) | $-CO_2Et$ | |
| 62 | 4-Cl | " | " | |
| 63 | 2,4-$Cl_2$ | " | " | |
| 64 | 3,5-$Cl_2$ | " | " | |
| 65 | 2,6-$Cl_2$ | " | " | |
| 66 | 4-$NO_2$ | " | " | |
| 67 | H | " | " | |
| 68 | 4-$OCH_3$ | " | " | |
| 69 | 2-$CH_3$ | " | " | |
| 70 | 2,4-$(CH_3)_2$ | " | " | |
| 71 | 4-F | " | " | |
| 72 | 3-$CF_3$ | " | " | |
| 73 | 2-Cl | $-OCH_3$ | $-C_6H_3$-2,4-$Cl_2$ | |
| 74 | 4-Cl | " | " | |
| 75 | 2,4-$Cl_2$ | " | " | |
| 76 | 3,5-$Cl_2$ | " | " | |
| 77 | 2,6-$Cl_2$ | " | " | |
| 78 | 4-$NO_2$ | " | " | |
| 79 | H | " | " | |
| 80 | 4-$OCH_3$ | " | " | |
| 81 | 2-$CH_3$ | " | " | |
| 82 | 2,4-$(CH_3)_2$ | " | " | |
| 83 | 4-F | " | " | |
| 84 | 3-$CF_3$ | " | " | |
| 85 | 2-Cl | $-OC_2H_5$ | " | |
| 86 | 4-Cl | " | " | |

(Forts. Tabelle I)

| Nr. | $(Z)_n{}^1$ | $-A-R^1$ | B | Schmp. °C |
|---|---|---|---|---|
| 87 | 2,4-$Cl_2$ | $-OC_2H_5$ | $-C_6H_3$-2,4-$Cl_2$ | |
| 88 | 3,5-$Cl_2$ | " | " | |
| 89 | 2,6-$Cl_2$ | " | " | |
| 90 | 4-$NO_2$ | " | " | |
| 91 | H | " | " | |
| 92 | 4-$OCH_3$ | " | " | |
| 93 | 2-$CH_3$ | " | " | |
| 94 | 2,4-$(CH_3)_2$ | " | " | |
| 95 | 4-F | " | " | |
| 96 | 3-$CF_3$ | " | " | |

**Biologische Beispiele**

**Beispiel 1**

Weizen und Gerste wurden im Gewächshaus in Plastiktöpfen bis zum 3-4 Blattstadium herangezogen und dann nacheinander mit den erfindungsgemäßen Verbindungen und den getesteten Herbiziden im Nachauflaufverfahren behandelt. Die Herbizide und die Verbindungen der Formel (I) wurden dabei in Form wäßriger Suspensionen bzw. Emulsionen mit einer Wasseraufwandmenge von umgerechnet 800 l/ha ausgebracht. 3-4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Die Bewertung erfolgte in Prozentwerten im Vergleich zu unbehandelten Kontrollen.

Die Ergebnisse aus Tabelle II veranschaulichen, daß die erfindungsgemäßen Verbindungen starke Herbizidschäden an den Kulturpflanzen effektiv reduzieren können.

Selbst bei starken Überdosierungen des Herbizids H werden bei den Kulturpflanzen auftretende schwere Schädigungen deutlich reduziert, geringere Schäden völlig aufgehoben. Mischungen aus Herbiziden und erfindungsgemäßen Verbindungen eignen sich deshalb in ausgezeichneter Weise zur selektiven Unkrautbekämpfung in Getreidekulturen.

Tabelle II

| Safenerwirkung der erfindungsgemäßen Verbindungen | | | |
|---|---|---|---|
| | | Kulturpflanzenschädigung (%) | |
| Bsp. Nr. | Dosis kg a.i./ha | TRAE | HOVU |
| H | 2,0<br>0,2 | 75<br>- | -<br>80 |
| H<br>+<br>3 | 2,0 + 2,5<br>0,2 + 2,5 | 40<br>- | -<br>35 |
| H<br>+<br>27 | 2,0 + 1,0<br>2,0 + 0,25<br>0,2 + 1,0<br>0,2 + 0.25 | 0<br>10<br>-<br>- | -<br>-<br>20<br>22 |
| Abkürzungen: | | | |
| TRAE = Triticum aestivum<br>HOVU = Hordeum vulgare<br>a.i. = Aktivsubstanz<br>H =<br>2-(4-(6-Chlorbenzoxazol-2-yloxy)phenoxypropionsäureeethylester (Fenoxaprop-ethyl) | | | |

## Ansprüche

1. Pflanzenschützende Mittel, dadurch gekennzeichnet, daß sie N-Arylhydrazone der allgemeinen Formel (I) oder deren Salze

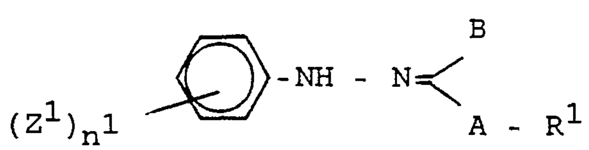

(I),

worin

$Z^1$ Halogen, Nitro, Cyano oder Alkyl, Alkoxy, Alkylthio oder Cycloalkyl, wobei die letzten vier genannten Reste unsubstituiert oder ein- oder mehrfach durch Alkoxy, Hydroxy oder Halogen substituiert sind, ferner Amino, Mono- oder Dialkylamino, Phenyl oder Phenoxy, wobei Phenyl oder Phenoxy unsubstituiert oder ein- oder mehrfach durch Halogen oder Halogenalkyl substituiert ist,

$R^1$ Alkyl, Alkenyl, Alkinyl oder Cycloalkyl, wobei die letztgenannten vier Reste unsubstituiert oder ein- oder mehrfach durch Reste aus der Gruppe Alkoxy, Alkylthio, Mono- oder Dialkylamino, Alkoxycarbonyl, Alkylcarbonyloxy, Cyano und Halogen substituiert sind, ferner Phenylalkyl oder Phenyl, die jeweils im Phenylrest unsubstituiert oder eine oder mehrfach durch Reste aus der Gruppe Alkyl, Alkoxy, Alkylthio, Mono- und Dialkylamino, Alkoxycarbonyl, Cyano und Halogen substituiert sind,

$n^1$ eine ganze Zahl von 0 bis 5 ist,

A O oder S,

B einen Rest der Formel

$-CO-R^2$ oder

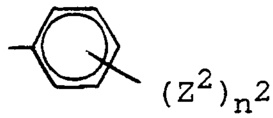,

worin
$Z^2$ die für $Z^1$ genannte Bedeutung hat und
$n^2$ eine ganze Zahl von 0 bis 5 ist, und
$R^2$ OH oder Alkoxy, Alkenyloxy, Alkinyloxy, Alkylthio oder Cycloalkoxy, wobei die fünf letztgenannten Reste unsubstituiert oder ein- oder mehrfach durch Reste aus der Gruppe Alkoxy, Alkylthio, Mono- und Dialkylamino, Cyano und Halogen substituiert sind,
ferner Trialkylsilylmethoxy, einen Rest der Formel

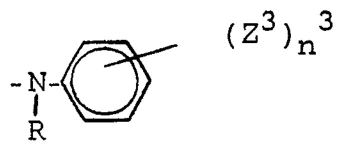 oder 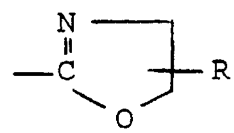,

worin jeweils R Wasserstoff oder Alkyl bedeutet, $Z^3$ die für $Z^1$ genannte Bedeutung hat und $n^3$ eine ganze Zahl von 0 bis 5 ist,
ferner Mono- oder Dialkylamino, Cycloalkylamino, Pyridino, Morpholino, Dimethylmorpholino, einen Rest der Formel

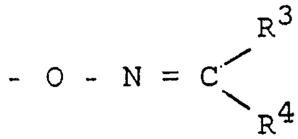,

worin $R^3$ und $R^4$ unabhängig voneinander Alkylreste bedeuten oder $R^3$ und $R^4$ gemeinsam mit dem sie verknüpfenden C-Atom einen Cycloalkylrest bilden,
bedeuten,
in Kombination mit üblichen Hilfsstoffen für Pflanzenschutzmittelformulierungen enthalten.

2. Pflanzenschützende Mittel nach Anspruch 1, dadurch gekennzeichnet, daß
$Z^1$ unabhängig voneinander Halogen, Nitro, Cyano, $(C_1$-$C_4)$Alkyl, $(C_1$-$C_4)$Alkoxy, $(C_1$-$C_4)$Alkylthio, wobei die Alkyl-, Alkoxy- und Alkylthiogruppen unsubstituiert oder ein-oder mehrfach durch Halogenatome, substituiert sind, $(C_3$-$C_6)$Cycloalkyl, das unsubstituiert oder durch $(C_1$-$C_4)$Alkyl substituiert ist, Amino, $(C_1$-$C_4)$Alkylamino, Di-$(C_1$-$C_4)$alkylamino, Hydroxymethyl, $(C_1$-$C_4)$Alkoxy-methyl, Phenyl oder Phenoxy, wobei Phenyl und Phenoxy unsubstituiert oder ein- oder mehrfach durch Halogen oder einfach durch Trifluormethyl oder durch ein oder mehrere Halogen und ein Trifluormethyl substituiert ist,
$n^1$ 0, 1, 2 oder 3,
A S oder 0,
$R^1$ $(C_3$-$C_6)$Cycloalkyl, $(C_1$-$C_6)$Alkyl, $(C_2$-$C_6)$Alkenyl, $(C_2$-$C_6)$Alkinyl, wobei die genannten vier Reste unsubstituiert oder ein- oder mehrfach durch Reste aus der Gruppe $(C_1$-$C_2)$Alkoxy, Mono- oder Di-$(C_1$-$C_6)$-Alkylamino, $(C_1$-$C_6)$Alkoxycarbonyl, $(C_1$-$C_6)$Alkylcarbonyloxy, $(C_1$-$C_2)$Alkylthio, Cyano und Halogen substituiert sind, ferner Phenyl-$(C_1$-$C_6)$alkyl oder Phenyl, die jeweils im Phenylrest unsubstituiert oder eine oder mehrfach durch Reste aus der Gruppe $(C_1$-$C_4)$Alkyl, $(C_1$-$C_4)$Alkoxy, $(C_1$-$C_4)$Alkylthio, Mono- und Di-$(C_1$-$C_6)$-alkylamino, $(C_1$-$C_6)$Alkoxy-carbonyl, Cyano, und Halogen substituiert sind,
B einen Rest der Formel
$-CO-R^2$ oder

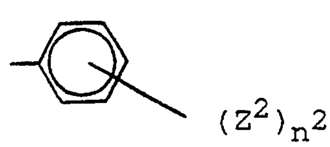,

worin

$Z^2$ die oben für $Z^1$ genannte Bedeutung hat und

$n^2$ 0, 1, 2 oder 3 ist,

$R^2$ Hydroxy, $-OCH_2Si(CH_3)_3$, $(C_3-C_6)$Cycloalkoxy, Phenyl$(C_1-C_6)$alkoxy, Phenoxy, $(C_2-C_6)$Alkenyloxy, $(C_2-C_6)$Alkinyloxy, $(C_1-C_6)$Alkoxy, $(C_1-C_6)$Alkylthio, wobei die Alkoxy oder Alkylthiogruppe unsubstituiert oder ein- oder zweifach durch $(C_1-C_2)$Alkoxy, Mono- oder Di-$(C_1-C_6)$Alkylamino, $(C_1-C_2)$Alkylthio, Cyano oder eine oder mehrfach durch Halogen substituiert ist,

einen Rest der Formel

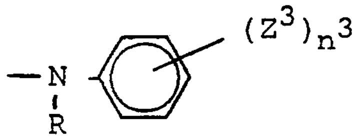 oder 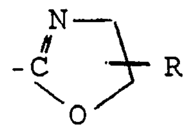 ,

worin jeweils R Wasserstoff oder $(C_1-C_4)$Alkyl bedeutet,

$Z^3$ die für $Z^1$ genannte Bedeutung hat und $n^3$ 0, 1, 2 oder 3 ist,

ferner Mono- oder Di-$(C_1-C_4)$Alkylamino, $(C_5-C_6)$Cycloalkylamino, Piperidino, Morpholino oder 2,6-Dimethylmorpholino, einen Rest der Formel

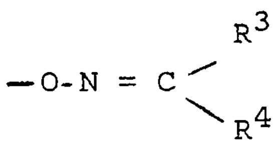 ,

worin

$R^3$ und $R^4$ unabhängig voneinander $(C_1-C_4)$Alkyl bedeuten oder $R^3$ und $R^4$ gemeinsam mit dem sie verknüpfenden C-Atom einen 5-, 6- oder 7-gliedrigen Cycloalkylrest bilden,

bedeutet.

3. Pflanzenschützende Mittel nach einem oder mehreren der Ansprüche 1 und 2, dadurch gekennzeichnet, daß

$Z^1$ unabhängig voneinander Halogen, insbesondere Fluor oder Chlor, Nitro, $(C_1-C_4)$Alkyl, $(C_1C_4)$Alkoxy oder Trifluormethyl,

A Sauerstoff,

$n^1$ 0, 1 oder 2,

$R^1$ Phenyl, das unsubstituiert oder ein- bis dreifach durch Reste aus der Gruppe $(C_1-C_4)$Alkyl, insbesondere Methyl, $(C_1-C_4)$Alkoxy und Halogen substituiert ist, oder $(C_1-C_4)$Alkyl,

B einen Rest der Formel

$-CO-R^2$ oder

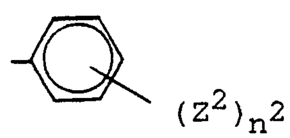 ,

worin

$R^2$ Hydroxy oder $(C_1-C_4)$Alkoxy,

$Z^3$ Halogen, insbesondere Chlor, oder $(C_1-C_4)$Alkyl und

$n^3$ 0, 1 oder 2 sind,

bedeuten.

4. Herbizides Mittel, dadurch gekennzeichnet, daß es mindestens eine Verbindung der Formel (I) nach einem oder mehreren der Ansprüche 1 bis 3 als Safener und ein Herbizid enthält.

5. Herbizides Mittel nach Anspruch 4, dadurch gekennzeichnet, daß es ein Herbizid aus der Gruppe der Carbamate, Thiocarbamate, Halogenacetanilide, substituierte Phenoxy-, Naphthoxy- und Phenoxyphenoxycarbonsäurederivate Heteroaryloxyphenoxycarbonsäurederivate und Dimedonabkömmlinge enthält.

6. Verfahren zum Schutz von Kulturpflanzen gegen phytotoxische Nebenwirkungen von Herbizide, dadurch gekennzeichnet, daß man die Pflanzen, Pflanzensamen oder Anbauflächen mit einer nach einem oder

mehreren der Ansprüche 1 bis 3 definierten Verbindung der Formel (I) vor, nach oder gleichzeitig mit dem Herbizid behandelt.

7. Verwendung einer nach einem oder mehreren der Ansprüche 1 bis 3 definierten Verbindung als Safener gegen phytotoxische Nebenwirkungen von Herbiziden.

8. Verfahren zur Bekämpfung von unerwünschtem Pflanzenwuchs dadurch gekennzeichnet, daß man die Pflanzen, Pflanzensamen oder Anbauflächen mit einer wirksamen Menge eines Herbizids in Kombination mit einer nach einem oder mehreren der Ansprüche 1 bis 3 definierten Verbindung der Formel (I) oder deren Salz behandelt.

9. Verbindungen der Formel (I) oder deren Salze,

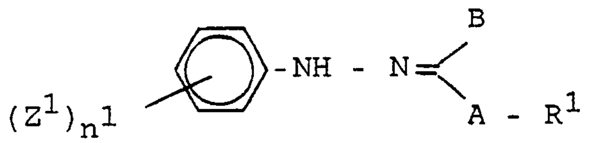

(I),

worin

$Z^1$, $R^1$, $n^1$, A und B, die nach einem oder mehreren der Ansprüche 1 bis 3 definierten Bedeutungen haben, mit Ausnahme solcher Verbindungen der Formel (I) oder deren Salze, worin

a) $R^1$ unsubstituiertes oder substituiertes Phenyl,
A Sauerstoff und
B einen Rest der oben definierten Formel

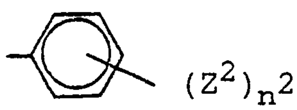

bedeuten, sowie mit Ausnahme solcher, bei denen

b) $Z^1$ 4-$CH_3$, 4-Br, 4-Cl, 4-$NO_2$, 3-$CH_3$,
$n^1$ 0 oder 1,
$R^1$ unsubstituiertes Phenyl, 4-Methylphenyl oder 3-Methylphenyl,
A Sauerstoff und
B $CO_2C_2H_5$
bedeuten.

10. Verfahren zur Herstellung von Verbindungen der Formel (I) oder deren Salze, die in Anspruch 9 definiert sind, dadurch gekennzeichnet, daß man eine Verbindung der Formel (II)

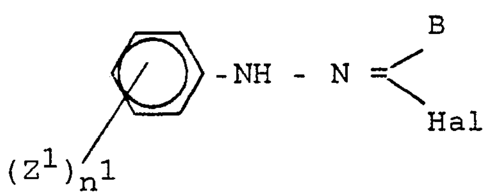

(II)

worin $(Z)_n1$ und B die oben angegebenen Bedeutungen besitzen und Hal, Br, Cl, J ist,
mit Alkoholen bzw. Thiolen der Formel HA-$R^1$
worin A und $R^1$ die oben genannten Bedeutungen haben,
und einer Base, wie z.B. NaOEt, $ROC_2H_5$, $NH_3$ und $NaOCOCH_3$,
umsetzt.

Patentansprüche für folgenden Vertragsstaat: ES

1. Verwendung von Verbindungen der Formel (I) oder deren Salze

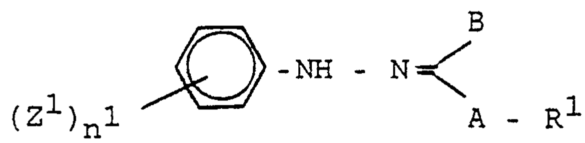 (I),

worin

$Z^1$ Halogen, Nitro, Cyano oder Alkyl, Alkoxy, Alkylthio oder Cycloalkyl, wobei die letzten vier genannten Reste unsubstituiert oder ein- oder mehrfach durch Alkoxy, Hydroxy oder Halogen substituiert sind, ferner Amino, Mono- oder Dialkylamino, Phenyl oder Phenoxy, wobei Phenyl oder Phenoxy unsubstituiert oder ein- oder mehrfach durch Halogen oder Halogenalkyl substituiert ist,

$R^1$ Alkyl, Alkenyl, Alkinyl oder Cycloalkyl, wobei die letztgenannten vier Reste unsubstituiert oder ein- oder mehrfach durch Reste aus der Gruppe Alkoxy, Alkylthio Mono- oder Dialkylamino, Alkoxycarbonyl, Alkylcarbonyloxy, Cyano und Halogen substituiert sind, ferner Phenylalkyl oder Phenyl, die jeweils im Phenylrest unsubstituiert oder ein- oder mehrfach durch Reste aus der Gruppe Alkyl, Alkoxy, Alkylthio, Mono- und Dialkylamino, Alkoxycarbonyl, Cyano und Halogen substituiert sind,

$n^1$ eine ganze Zahl von 0 bis 5 ist,

A O oder S,

B einen Rest der Formel

-CO - $R^2$ oder

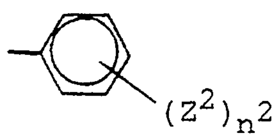 ,

worin $Z^2$ die für $Z^1$ genannte Bedeutung hat und

$n^2$ eine ganze Zahl von 0 bis 5 ist, und

$R^2$ OH oder Alkoxy, Alkenyloxy, Alkinyloxy, Alkylthio oder Cycloalkoxy, wobei die fünf letztgenannten Reste unsubstituiert oder ein- oder mehrfach durch Reste aus der Gruppe Alkoxy, Alkylthio, Mono- und Dialkylamino, Cyano und Halogen substituiert sind,

ferner Trialkylsilylmethoxy, einen Rest der Formel

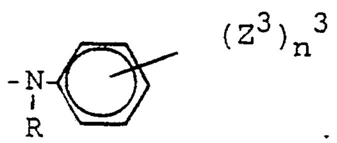 . oder 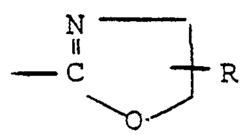 ,

worin jeweils R Wasserstoff oder Alkyl bedeutet, $Z^3$ die für $Z^1$ genannte Bedeutung hat und $n^3$ eine ganze Zahl von 0 bis 5 ist,

ferner Mono- oder Dialkylamino, Cycloalkylamino, Pyridino, Morpholino, Dimethylmorpholino, einen Rest der Formel

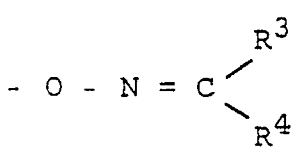 ,

worin $R^3$ und $R^4$ unabhängig voneinander Alkylreste bedeuten oder $R^3$ und $R^4$ gemeinsam mit dem sie verknüpfenden C-Atom einen Cycloalkylrest bilden,

bedeuten,

als pflanzenschützende Mittel gegen phytotoxische Nebenwirkungen von Herbiziden.

2. Verwendung nach Anspruch 1, dadurch gekennzeichnet, daß

$Z^1$ unabhängig voneinander Halogen, Nitro, Cyano, $(C_1-C_4)$Alkyl, $(C_1-C_4)$Alkoxy, $(C_1-C_4)$Alkylthio, wobei die Alkyl-, Alkoxy- und Alkylthiogruppen unsubstituiert oder ein-oder mehrfach durch Halogenatome,

substituiert sind, $(C_3\text{-}C_6)$Cycloalkyl, das unsubstituiert oder durch $(C_1\text{-}C_4)$Alkyl substituiert ist, Amino, $(C_1\text{-}C_4)$Alkylamino, Di-$(C_1\text{-}C_4)$alkylamino, Hydroxymethyl, $(C_1\text{-}C_4)$Alkoxy-methyl, Phenyl oder Phenoxy, wobei Phenyl und Phenoxy unsubstituiert oder ein- oder mehrfach durch Halogen oder einfach durch Trifluormethyl oder durch ein oder mehrere Halogen und ein Trifluormethyl substituiert ist,

$n^1$ 0, 1, 2 oder 3,

A S oder 0,

$R^1$ $(C_3\text{-}C_6)$Cycloalkyl, $(C_1\text{-}C_6)$Alkyl, $(C_2\text{-}C_6)$Alkenyl, $(C_2\text{-}C_6)$Alkinyl, wobei die genannten vier Reste unsubstituiert oder ein- oder mehrfach durch Reste aus der Gruppe $(C_1\text{-}C_2)$Alkoxy, Mono- oder Di-$(C_1\text{-}C_6)$-Alkylamino, $(C_1\text{-}C_6)$Alkoxycarbonyl, $(C_1\text{-}C_6)$Alkylcarbonyloxy, $(C_1\text{-}C_2)$Alkylthio, Cyano und Halogen substituiert sind, ferner Phenyl-$(C_1\text{-}C_6)$alkyl oder Phenyl, die jeweils im Phenylrest unsubstituiert oder eine oder mehrfach durch Reste aus der Gruppe $(C_1\text{-}C_4)$Alkyl, $(C_1\text{-}C_4)$Alkoxy, $(C_1\text{-}C_4)$Alkylthio, Mono- und Di-$(C_1\text{-}C_6)$-alkylamino, $(C_1\text{-}C_6)$Alkoxy-carbonyl, Cyano, und Halogen substituiert sind,

B einen Rest der Formel

$-CO-R^2$ oder

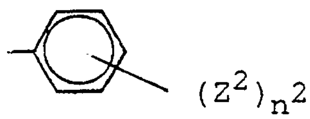 ,

worin

$Z^2$ die oben für $Z^1$ genannte Bedeutung hat und

$n^2$ 0, 1, 2 oder 3 ist,

$R^2$ Hydroxy, $-OCH_2Si(CH_3)_3$, $(C_3\text{-}C_6)$Cycloalkoxy, Phenyl$(C_1\text{-}C_6)$alkoxy, Phenoxy, $(C_2\text{-}C_6)$Alkenyloxy, $(C_2\text{-}C_6)$Alkinyloxy, $(C_1\text{-}C_6)$Alkoxy, $(C_1\text{-}C_6)$Alkylthio, wobei die Alkoxy oder Alkylthiogruppe unsubstituiert oder ein- oder zweifach durch $(C_1\text{-}C_2)$Alkoxy, Mono- oder Di-$(C_1\text{-}C_6)$Alkylamino, $(C_1\text{-}C_2)$Alkylthio, Cyano oder ein-oder mehrfach durch Halogen substituiert ist,

einen Rest der Formel

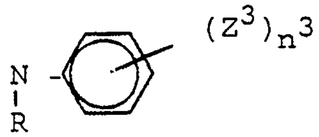 oder 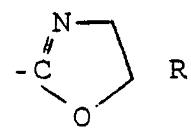 ,

worin jeweils R Wasserstoff oder $(C_1\text{-}C_4)$Alkyl bedeutet,

$Z^3$ die für $Z^1$ genannte Bedeutung hat und $n^3$ 0, 1, 2 oder 3 ist,

ferner Mono- oder Di-$(C_1\text{-}C_4)$Alkylamino, $(C_5\text{-}C_6)$Cycloalkylamino, Piperidino, Morpholino oder 2,6-Dimethylmorpholino, einen Rest der Formel

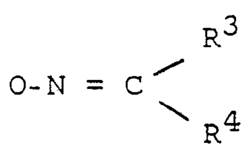 ,

worin

$R^3$ und $R^4$ unabhängig voneinander $(C_1\text{-}C_4)$Alkyl bedeuten oder $R^3$ und $R^4$ gemeinsam mit dem sie verknüpfenden C-Atom einen 5-, 6- oder 7-gliedrigen Cycloalkylrest bilden,

bedeutet.

3. Verwendung nach Anspruch 1 oder 2, dadurch gekennzeichnet, daß

$Z^1$ unabhängig voneinander Halogen, insbesondere Fluor oder Chlor, Nitro, $(C_1\text{-}C_4)$Alkyl, $(C_1\text{-}C_4)$Alkoxy oder Trifluormethyl,

A Sauerstoff,

$n^1$ 0, 1 oder 2,

$R^1$ Phenyl, das unsubstituiert oder ein- bis dreifach durch Reste aus der Gruppe $(C_1\text{-}C_4)$Alkyl, insbesondere Methyl, $(C_1\text{-}C_4)$Alkoxy und Halogen substituiert ist, oder $(C_1\text{-}C_4)$Alkyl,

B einen Rest der Formel

-CO - $R^2$ oder

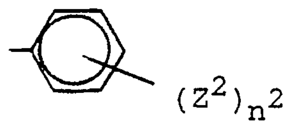

,

worin
$R^2$ Hydroxy oder $(C_1\text{-}C_4)$Alkoxy,
$Z^3$ Halogen, insbesondere Chlor, oder $(C_1\text{-}C_4)$Alkyl und
$n^3$ 0, 1 oder 2 sind,
bedeuten.

4. Verwendung nach einem oder mehreren der Ansprüche 1 bis 3, dadurch gekennzeichnet, daß ds Herbizid aus der Gruppe der Carbamate, Thiocarbonate, Halogenacetanilide, substituierte Phenoxy-, Naphtoxy- und Phenoxyphenoxycarbonsäurederivate Heteroaryloxyphenoxycarbonsäurederivate und Dimedonabkömmlinge ausgewählt ist.

5. Verfahren zum Schutz von Kulturpflanzen gegen phytotoxische Nebenwirkungen von Herbizide, dadurch gekennzeichnet, daß man die Pflanzen, Pflanzensamen oder Anbauflächen mit einer nach einem oder mehreren der Ansprüche 1 bis 3 definierten Verbindung der Formel (I) vor, nach oder gleichzeitig mit dem Herbizid behandelt.

6. Verfahren zur Bekämpfung von unerwünschtem Pflanzenwuchs dadurch gekennzeichnet, daß man die Pflanzen, Pflanzensamen oder Anbauflächen mit einer wirksamen Menge eines Herbizids in Kombination mit einer nach einem oder mehreren der Ansprüche 1 bis 3 definierten Verbindung der Formel (I) oder deren Salz behandelt.

7. Verfahren zur Herstellug von Verbindungen der Formel (I) oder deren Salze

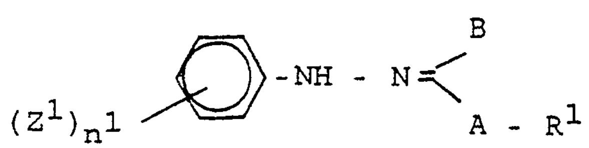

(I),

worin
$Z^1$, $R^1$, $n^1$, A und B, die nach einem oder mehreren der Ansprüche 1 bis 3 definierten Bedeutungen haben, mit Ausnahme solcher Verbindungen der Formel (I) oder deren Salze, worin
a) $R^1$ unsubstituiertes oder substituiertes Phenyl,
A Sauerstoff und
B einen Rest der oben definierten Formel

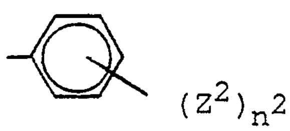

bedeuten, sowie mit Ausnahme solcher, bei denen
b) $Z^1$ 4-$CH_3$, 4-Br, 4-Cl, 4-$NO_2$, 3-$CH_3$,
$n^1$ 0 oder 1,
$R^1$ unsubstituiertes Phenyl, 4-Methylphenyl oder 3-Methylphenyl,
A Sauerstoff und
B $CO_2C_2H_5$
bedeuten,
dadurch gekennzeichnet, daß man eine Verbindung der Formel (II)

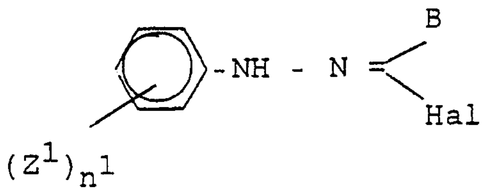

(II)

worin $(Z)_n1$ und B die oben angegebenen Bedeutungen besitzen und Hal, Br, Cl, J ist, mit Alkoholen bzw. Thiolen der Formel HA-R[1] worin A und R die oben genannten Bedeutungen haben, und einer Base, wie z.B. NaOEt, $ROC_2H_5$, $NH_3$ und $NaOCOCH_3$, umsetzt.